# EUROPEAN PATENT APPLICATION

(11) **EP 1 452 147 A1**
(43) Date of publication of application: **01.09.2004**
(21) Application number: 04001964.8
(22) Date of filing: 29.01.2004
(51) Int. Cl.: A61B 19/00

(54) **Surgical operation assistance system**

(30) Priority: 26.02.2003 JP 2003048708
(71) Applicant: Hitachi, Ltd., Chiyoda-ku, Tokyo 101-8010 (JP)
(72) Inventor: Shose, Ako, Chiyoda-ku Tokyo 100-8220 (JP); Kan, Kazutoshi, Chiyoda-ku Tokyo 100-8220 (JP); Momoi, Yasuyuki, Chiyoda-ku Tokyo 100-8220 (JP)
(74) Representative: Beetz & Partner Patentanwälte

(57) **Abstract**

The present invention relates to a system for surgical operation assistance. A surgical operation assistance system (4) picks up an image of a surgical field using an image pick-up device (100). An image producing unit (101) produces a stereographic image of the surgical field, the image of which is picked up. Reference points of a surgical operation route are inputted based on the kind of the surgical operation and the stereographic image. A surgical operation route calculation unit (107) calculates a smooth surgical operation route based on the kind of the surgical operation and the reference points inputted, to be displayed on an image displaying apparatus (104).

## Description

### BACKGROUND OF THE INVENTION

The present invention relates to an operation assistance system, and in particular, it relates to a surgical operation assistance system, being suitable for assistance of surgical operations, particularly orthopedic operations, with using a computer system therein.

In recent years, accompanied with the development of imaging devices for picking up images for medical use, a method has been widely spread for planning surgical operations by referring to images picked up for the medical use (hereinafter called "medical use images"), which are displayed on a computer. Accompanied therewith, an apparatus has been developed for assisting a surgical operation plan on a computer.

An example of such a conventional technology is described in, for example, JP 2000-113086 (2000), JP 2001-293007 (2001), and JP-Hei 11-155881 (1999). The surgical operation assistance system described in those publications comprises: An image picking-up apparatus for picking up a surgical field; an image producing portion for producing a stereoscopic image (i.e. a three-dimensional vision) of the surgical field, which is picked up; a surgical operation route calculating portion for calculating a route for the surgical operation; and an image display apparatus for displaying the stereoscopic images and the surgical operation route thereon.

Such an operation assistance system described in each of the publications mentioned above is, however, mainly used in cerebral surgical operations, for the purpose of obtaining an operation route of low invasion, up to the surgical field, using the medical use image of a patient, which is picked up. However, the possibility of using such a system in orthopedic surgical operations in the surgical field, such as, the ostectomy, for example, has not been taken into consideration. In particular, in orthopedic surgical operations, it is necessary to determine a smooth operation route relating to the surgical field, however, the surgical operation assistance systems described in those publications mentioned above cannot cope with such a purpose.

### BRIEF SUMMARY OF THE INVENTION

It is an object of the present invention to provide a surgical operation assistance system and a surgical operation assisting method, and also a program thereof, allowing an easy determination of a smooth operation route up to the surgical field, and thereby enabling particularly orthopedic surgical operations to be carried out with ease and security.

The above problem is solved according to the independent claims. The dependent claims relate to preferred embodiments of the concept of the present invention.

For accomplishing the object mentioned above, according to the present invention, there is provided a surgical operation assistance system, comprising: an image pick-up apparatus for picking up an image of a surgical field; an image producing unit for producing a stereographic image of the surgical field, the image of which is picked up; an input unit for inputting reference points of a surgical operation route on the basis of the kind of the surgical operation and the stereographic image; a surgical operation route calculation unit for calculating a smooth surgical operation route on the basis of the kind of the surgical operation inputted and the reference points; an image processing unit for processing the stereographic image and the surgical operation route to be displayable; and an image displaying apparatus for displaying the images processed in the image processing unit thereon.

Herein, according to the present invention, more preferably, the structure of the surgical operation assistance system as described in the above is as follows. Thus, it further comprises an image extracting unit for extracting a partial image from the stereographic image, wherein the image processing unit processes the extracted image to be displayable. Also, it further comprises a slice image arbitrary line input unit for designating an arbitrary line of a sliced image to be displayed, wherein the image processing unit processes the sliced image to be displayable, on the basis of the arbitrary line designated for the slice image. And, also, it further comprises a surgical operation robot for automatically conducting the surgical operation on the surgical field with using the surgical operation tool along with the surgical operation route calculated.

Further, for solving the problem mentioned above, according to the present invention, there is also provided a surgical operation assistance system, comprising: an image pick-up apparatus for picking up an image of a surgical field; a surgical operation robot for conducting a surgical operation on the surgical field using a surgical operation tool; a position information integration unit for integrating position information of the surgical operation robot with an image of the surgical field picked up by the image pick-up -apparatus; an image producing unit for producing a stereographic image of the surgical field picked up, and for producing an image piling up an image of the surgical operation tool on the stereographic image, based on the information integrated in the position information integration unit; a reference point inputting unit for inputting reference points of a surgical operation route on the basis of the kind of the surgical operation tool and the stereographic image; a surgical operation route calculating unit for calculating a smooth surgical operation route on the basis of the kind of the surgical operation tool and the reference points, which are inputted; an image processing unit for processing the stereographic image and the surgical operation route to be displayable under desired conditions; and an image displaying apparatus for displaying thereon the image processed in the image processing unit.

Herein, according to the present invention, more preferably, in the surgical operation assistance system as described above, the surgical operation robot is also operable on the surgical field manually, with using the surgical operation tool along with the surgical operation route calculated, and the surgical operation robot is switchable or changeable between automatic operation and manual operation thereof.

And, for solving the problem mentioned above, according to the present invention, there is further provided as surgical operation assisting method, comprising the following steps: picking up an image of a surgical field by means of an image pick-up apparatus; producing a stereographic image of the surgical field, the image of which is picked up, in an image producing unit; inputting reference points of a surgical operation route on the basis of the kind of the surgical operation and the stereographic image through an input unit; calculating a smooth surgical operation route based on the kind of the surgical operation inputted and the reference points in a surgical operation route calculation unit; processing the stereographic image and the surgical operation route to be displayable in an image processing unit; and displaying the images processed in the image processing unit on an image displaying apparatus.

And also, for solving the problem mentioned above, according to the present invention, there is further provided a surgical operation assisting method, comprising the following steps: picking up an image of a surgical field by means of an image pick-up apparatus; conducting a surgical operation on the surgical field manually, with using a surgical operation tool of a surgical operation robot; integrating position information of the surgical operation robot with an image of the surgical field picked up by the image pick-up apparatus in a position information integration unit; producing a stereographic image of the surgical field picked up, and for producing an image piling up an image of the surgical operation tool on the stereographic image, on the basis of the information integrated in the position information integration unit, in an image producing unit; inputting reference points of a surgical operation route on the basis of a kind of the surgical operation tool and the stereographic image through a reference point inputting unit; calculating a smooth surgical operation route based on the kind of the surgical operation tool and the reference points, which are inputted, in a surgical operation route calculating unit; processing the stereographic image and the surgical operation route to be displayable under a desired condition in an image processing unit; and displaying the image processed in the image processing unit on an image displaying apparatus.

Herein, more preferably, according to the present invention, in the surgical operation assisting method as described in the above, the image of the surgical field is picked up by means of the image pick-up apparatus under a condition where markers are attached thereon in a number of three (3) or more, thereby producing a medical use image, further comprising the following steps: attaching the same number of markers on an actual patient at positions where the markers are attached, measuring position coordinates of those markers through a three-dimension position measuring apparatus, thereby presenting them in a form of a matrix of 3x3 or more; converting this matrix into a matrix of 3x3 or more for presenting the position coordinates of the markers on the medical use image in the position information integration unit; and producing an image piling up the image of the surgical operation tool on the stereographic image, upon the converted matrix.

And, also for solving the problem mentioned above, according to the present invention, there is further provided a program stored on a computer-readable storage medium for assisting particularly orthopedic surgical operations, comprising the following steps: a step of picking up an image of a surgical field by means of an image pick-up apparatus; a step of producing a stereographic image of the surgical field, the image of which is picked up, in an image producing unit; a step of inputting reference points of a surgical operation route on the basis of the kind of the surgical operation and the stereographic image through an input unit; a step of calculating a smooth surgical operation route based on the kind of the surgical operation inputted and the reference points in a surgical operation route calculation unit; a step of processing the stereographic images and the surgical operation route to be displayable in an image processing unit; and a step of displaying the images processed in the image processing unit on an image displaying apparatus.

### BRIEF DESCRIPTION OF THE DRAWINGS

Those and other objects, features and advantages of the present invention will become more readily apparent from the following detailed description when taken in conjunction with the accompanying drawings, wherein:
Fig. 1 is a block diagram of showing an embodiment of a surgical operation assistance system according to the present invention;
   and
Figs. 2 to 6 show screen images displayed on a display apparatus of the surgical operation assistance system shown in Fig. 1 mentioned above, wherein:

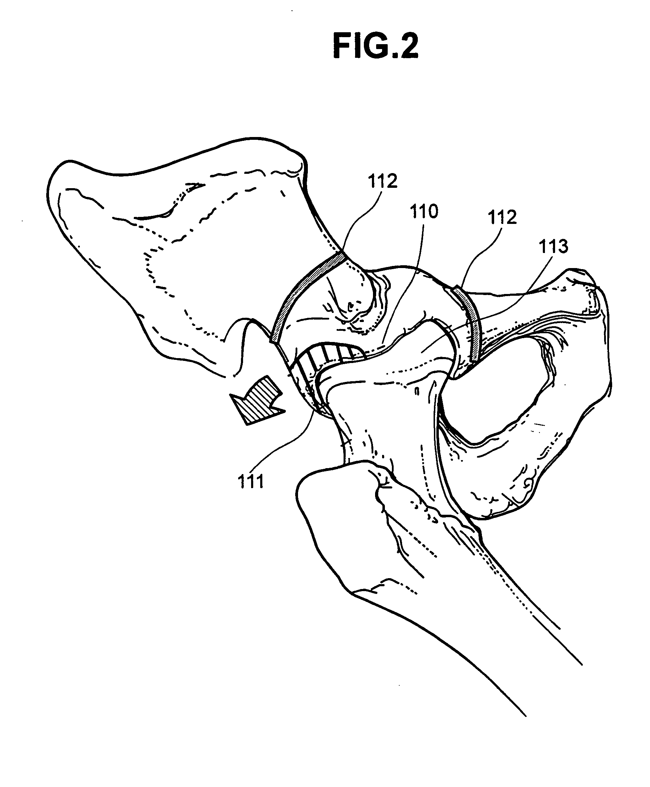

   Fig. 2 shows an example of the screen image of the surgical field;

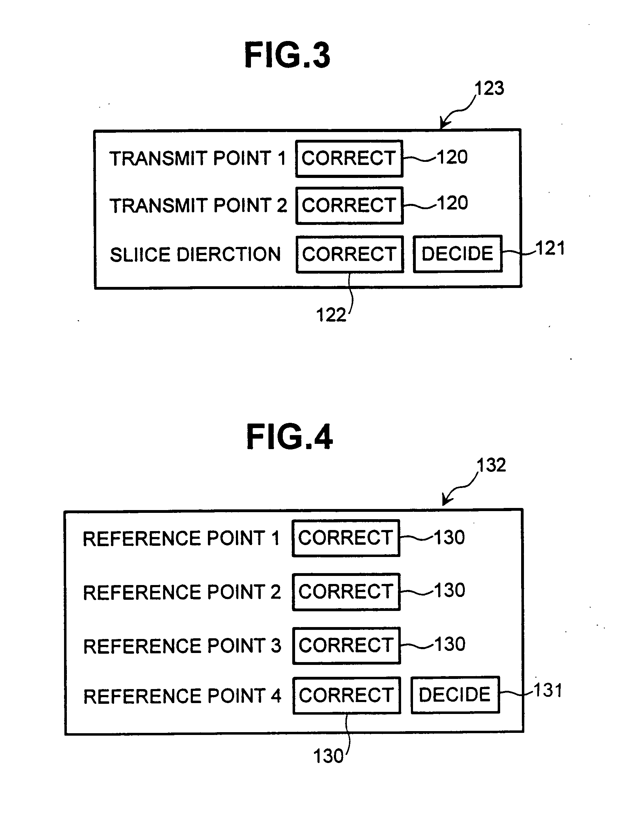

   Fig. 3 shows an example of a dialog screen of an arbitrary line input unit for use of showing a slice screen;
   Fig. 4 shows an example of a dialog screen of a reference point input unit;

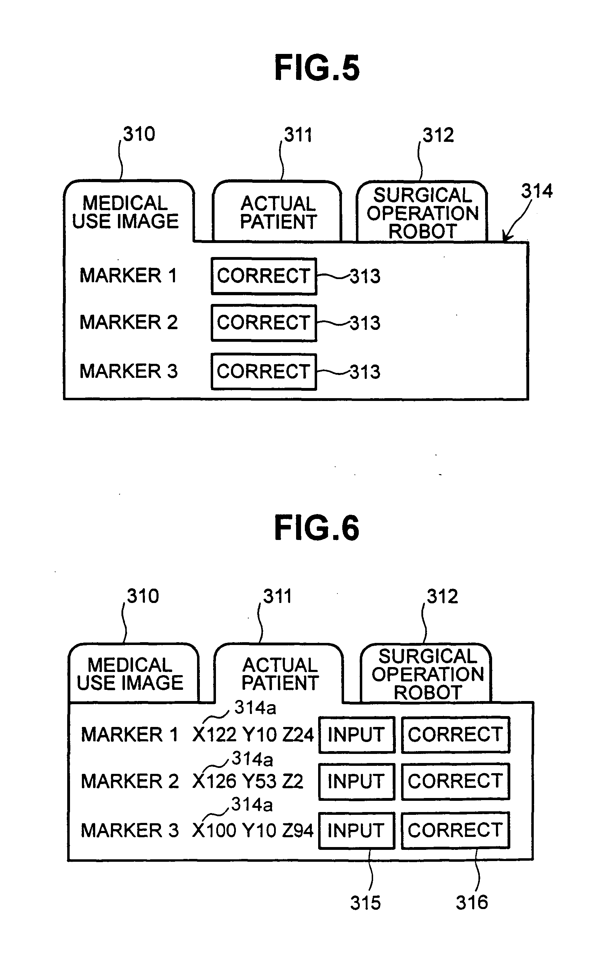

   Fig. 5 shows an example of a dialog screen of a positional information integration unit; and
   Fig. 6 shows an example of another dialog screen.

### DETAILED DESCRIPTION OF THE INVENTION

Hereinafter, embodiments according to the present invention will be fully explained by referring to the attached drawings.

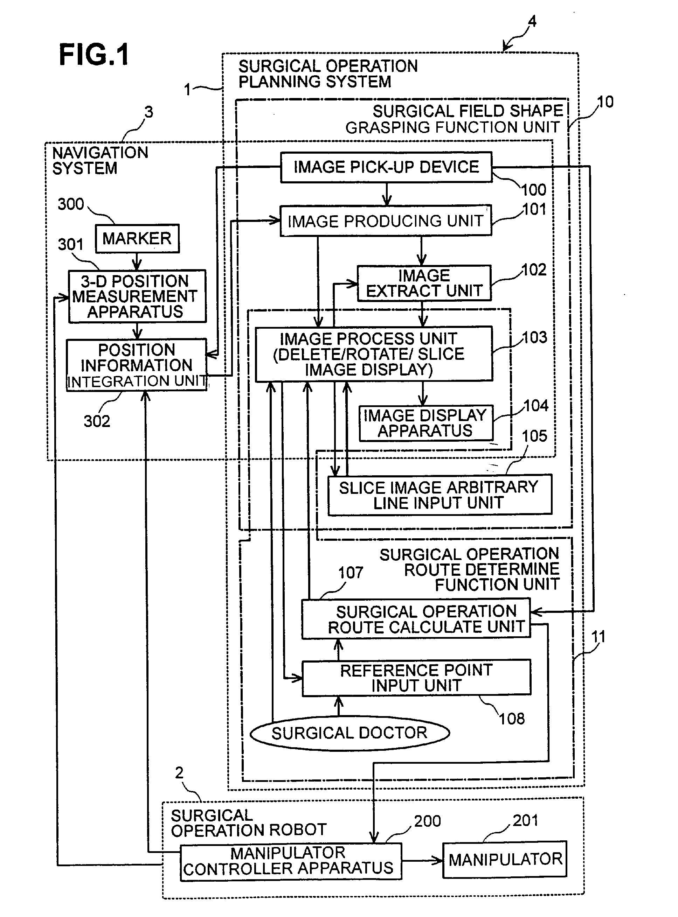

Hereinafter, explanation will be given of a first embodiment of the present invention, by referring to Figs. 1 to 6. The present embodiment will be explained by an example, in particular relating to the case of conducting a surgical operation through Rotating Acetabular Ostectomy (hereinafter, being abbreviated as RAO), with applying a surgical operation robot therein.

First of all, explanation will be given by referring to Fig. 2 attached herewith, in relation to the RAO. Fig. 2 is a view showing bones of a coax (i.e., a hip joint), and it is a view described in the document "Surgical Exposures in Orthopedics", Stanley Md. Hopperfeld, et al., in particular, on page 344 of the Japanese translation, edited by Tera-yama et al. (1998).

In a case, when the acetabular roof 110 of the coax does not cover the upper part of the caput ossis femoris in the shape thereof, in a patient to whom RAO is applied, due to hypoplasia, thereby causing a defective portion 111 in the condition thereof, for example, the body weight rests on it, under the condition that the caput ossis femoris 113 projects a little bit outside the acetabular roof 110. For this reason, the force applying on the acetabular roof 110 rests, not being distributed over the entire surface thereof, but being concentrated at an edge thereof, on the central side of the body, and therefore, the patient feels pains every time when she/he takes a step.

The RAO is such a kind of surgical operation wherein the acetabular roof 110 is cut off at the portion which is surrounded by an ostectomy line (or bone cutting line) 112 shown by a thick line in the Figure, thereby rotating the portion surrounded by the bone cutting line 112 in the direction of the arrow, so that the upper part of the caput ossis femoris 113 can be covered by the entire of the acetabular roof 110.

In the present embodiment, it is assumed that the orthopedic surgical operation is done with using a surgical operation robot, so that the bone is cut smoothly into a spherical shape in accordance with the surgical operation plan made up, and that the cutting portion of the surgical operation is small in the range thereof, thereby enabling the patient to move independently early after having the surgical operation, with commonly using a brace outside the wound (i.e., an assisting tool for enabling the patient to walk before fixation of the bones which are connected), and it relates to a surgical operation assistance system being necessary in such a case as mentioned above.

Next, explanation will be given on the surgical operation assistance system, according to the present embodiment, by referring to Fig. 1. Fig. 1 is a block diagram view showing the surgical operation assistance system.

The surgical operation assistance system 4 comprises a surgical operation planning system 1, a surgical operation robot 2, and a navigation system 3. This surgical operation system 4 comprises a computer and various kinds of input devices, and also output devices, and so on. The surgical operation planning system 1 comprises a surgical field shape grasping function unit 10 for grasping the condition of the surgical field, and a surgical operation route determining function unit 11 for determining the surgical operation route, fitting to the condition of the surgical field. This surgical operation planning system 1 is constructed such that it grasps the condition of the surgical field by means of the surgical field shape grasping function unit 10, and determines the suitable surgical operation route fitting to the determined condition of the surgical operation route, thereby checking the optimization of the surgical operation plan on the basis thereof, in the surgical field shape grasping function unit 10.

The computer comprises an image pick-up device 100, an image producing unit 101, an image extracting unit 102, an image processing unit 103, an image displaying apparatus 104 for displaying the image thereon, an arbitrary line input unit 105 for use of showing a sliced image, a reference point input unit 108, a surgical operation route calculation unit 107, and a positional information integration unit 302. Those constituent elements are built up with programs, and also are executed in accordance with operation steps which will be explained later. Further, the computer may be divided into a plural number of portions thereof, particularly functional portions.

The surgical operation assistance system 4 is a system for making up a plan for the surgical operation using the surgical operation planning system 1, before actually conducting the surgical operation, and it is that, during the surgical operation, for indicating or presenting the positional information of the surgical operation robot 2 with respect to the patient, to a surgical doctor, as well as the surgical operation plan, laying it on the medical use image of the patient. In the present embodiment, the surgical operation planning system 1 and the navigation system 3 are built up, by using portions thereof in common.

The surgical operation planning system 1 comprises the surgical field shape grasping function portion 10 for grasping the condition of the patient, and the surgical operation route determining function unit 11 for determining the surgical operation route fitting to the condition of the surgical field. This surgical operation planning system 1 is constructed such that the condition of the surgical field is grasped in the surgical field shape grasping function unit 10, that the suitable surgical operation route is determined fitting to the condition of the surgical field in the surgical operation route determining function unit 11, and on the basis of those, the optimization of the surgical operation plan is checked in the surgical field shape grasping function unit 10.

The surgical field shape grasping function unit 10 comprises the image pick-up device 100 for picking up an image of the surgical field, the image producing unit 101 for producing a slice image and/or a stereographic image, and the image extracting unit 102 for extracting an image of the necessary portion from the produced images, the image processing unit 103 for processing the extracted image so that it can be displayed under a desired condition thereof, the image displaying apparatus 104 for displaying the image thereon, and the arbitrary line input unit 105 for use of the slice image, for designating a sliced image to be seen.

This surgical field shape grasping function unit 10 picks up the medical use image in the vicinity of the surgical field through the image pick-up device 100, and reads a film of the medical use image, which is picked up, into the image producing unit 101, thereby producing the sliced image and/or the stereographic image thereof. And then, only the image of a portion of the bone is extracted from the stereographic image produced in the image extracting unit 102, and it is processed in the image processing unit 103, so that the stereographic image of the bone extracted can be displayed, rotatably. Thus, in the structure mentioned above, this image is presented to the surgical doctor on the image display apparatus 104. With this, the surgical doctor can check the shape of the bone from various angles thereof.

Further, the image pick-up device 100 is an instrument for picking up the image of the surgical field, such as, an image of an endoscope, an image of an electron microscope, an image picked-up through a CCD camera, an MRI or CT image, an ultrasonic image, and others. The image displaying apparatus 104 is an instrument for displaying the image information, which is transmitted from the image processing unit 103, on a CRT, a head mount display or other image display devices, or alternatively, that of directly projecting the image information on the patient.

In a case where it is desired to check the configuration of the bone on a sliced cross-section view thereof, so as to inspect the conditions of the acetabular roof, in more detail, for example, in the planning of a surgical operation, the surgical doctor designates the slice image that she/he wishes to see through the arbitrary line input unit 105 for use of slice images. After being processed in the image processing unit 103, then the image is presented to the surgical doctor in the form of the sliced image on the image display apparatus 104. This enables the surgical doctor to make up a suitable surgical operation plan.

When designating the sliced image through the arbitrary line input unit 105 for the slice image, "Sliced Cross-Section Display by Arbitrary Line" is selected from a menu screen displayed on the image display apparatus 104. With this, the screen of presenting the stereographic view of the surgical field appears on the image display apparatus 104, and also a dialog screen 123 appears, but at the end of that screen, as shown in Fig. 3.

Then, when clicking at an arbitrary point on the stereographic image of the surgical field through a mouse, the color of that point is changed, thereby designating a first passage point of the arbitrary line for use of the sliced image. However, when correcting that passage point, it is possible to alter the passage point to a new correction point, through clicking of the mouse at the correction point on the screen presenting the stereographic image of the surgical field, after pushing down a correct button 120 at the point corresponding thereto on the dialog screen 123. When designating a second passage point of the arbitrary line for use of the sliced image, after designating the first passage point, the mouse-click is made, but without pushing down the correct button 120. With this, the point where the mouse-click is made is automatically acknowledged to be the second passage point. When designating the passage point, it is possible to designate it while rotating the stereographic image of the surgical field, freely, through dragging of the mouse.

When the second passage point is designated, a sliced image passing through the two (2) points, in the vertical direction, is displayed, in the place of the image presenting the stereographic image thereof. If the direction of this sliced image is improper, the direction of this sliced image can be changed through moving it up to a desired sliced image, through dragging of the mouse. Herein, when pushing down a decide button 121 on the dialog screen 123, then the cross-section of the sliced screen is displayed thereon. This enables the surgical doctor to make up the suitable plan for the surgical operation.

However, when pushing down a correct button 122 of the slice direction on the dialog screen 123, it is possible to designate the direction of this sliced image, again, starting from the timing of determining thereof. Also, when pushing down the correct button 120 of the passage point 1 or 2 on the dialog screen 123, it is possible to designate that passage point, again, starting from the timing of determining thereof.

The surgical operation route determining function unit 11 comprises the reference point input unit 108 for inputting a reference of the surgical operation route, the surgical operation route calculation unit 107 for calculating the surgical operation route on the basis of the reference inputted, and a surgical operation route confirming unit, on which the surgical operation route calculated is displayed. The surgical operation route confirming unit is built up by using the image processing unit 103 and the image displaying apparatus 104 in common, as is shown in Fig. 1.

The surgical operation route determining function unit 11 has the functions: such as, of calculating a spherical surface passing through the reference point inputted in the surgical operation route calculating unit 107, after the surgical doctor designates "Determine Ostectomy Line" among the kinds of surgical operations from the menu-bar displayed on the image displaying apparatus 104 and "Spherical Surface" among the kinds of surfaces and lines for approximating the surgical operation route, and also she/he inputs the reference points corresponding to that kind which is designated (e.g., four (4) points, in this case, because of the spherical surface) through the reference point input unit 108; and, of presenting an intersection line between the spherical surface calculated and the bone on the image display apparatus 104 of the surgical operation route confirming unit. However, the surgical operation route determining function unit 11 may describe a curved surface, smoothly, passing through the reference points inputted, like drawing software, through inputting the number of the reference points which she/he wishes to input from a list screen of the menu-bar, after the surgical doctor designates the "Determine Ostectomy Line" among the kinds of surgical operations from the menu-bar, thereby enabling to present it to the surgical doctor on the image displaying apparatus 104 of the surgical operation route confirming unit.

When inputting the reference points through the reference point input unit 108, since a dialog screen 132, such as shown in Fig. 4, appears on an end portion on the screen through inputting of the kind of surgical operation and the approximating surface of the surgical operation from the menu-bar; therefore, the first reference point is inputted using this screen. The inputting of the reference point via the reference point input unit 108 is carried out, through dragging of the mouse, so as to freely rotate the stereographic image displaying an arbitrary point to be inputted as the reference point, and also clicking of the mouse at that arbitrary point. In this instance, the point at which the input is made changes in the color thereof; therefore it is possible to confirm easily that the input is made. However, when correction is made on the reference point, after pushing down the correct button 130 at the point corresponding thereto on the dialog screen 132, the clicking of the mouse is made at the reference point on the stereographic image, again, thereby achieving the change of the reference point to a new correction point. When designating the second point and also others following thereafter, after designating the last reference point (i.e., the first one), the mouse is clicked, but without pushing down the correct button 130. With this, the point at which the mouse is clicked is automatically acknowledged to be the next passing point.

When a decide button 131 is pushed down on the dialog screen shown in Fig. 4 by the surgical doctor, after completing the inputs of all the four (4) references points, the Ostectomy Line is calculated in the surgical operation route calculation unit 107. The surgical operation route calculation unit 107 calculates an equation of the spherical surface in the coordinate system of the medical use image for presentation, passing through all of the four (4) references points, and it further determines whether the numerical values of the medical use image data on the equation of the spherical surface represents the bone or not, thereby calculating the Ostectomy line on the surface where the equation of the spherical surface comes crossing the bone. After being converted into the image information in the image processing unit 103, the Ostectomy Line is presented to the surgical doctor on the image displaying device 104.

Study of the Ostectomy Line is conducted while simulating the surgical operation in the image processing unit 103, e.g., by rotating the bone surrounded by the Ostectomy Lines through dragging of the mouse with pushing down the other button, being opposite to that used for rotation of the entire stereographic image, in the horizontal direction thereof. In that instance, the surgical doctor can make confirmation at an arbitrary timing on the relationship between the Ostectomy Lines and an important organ therearound; e.g., on whether the important organ would be injured or not when rotating the bone, or on whether it is difficult or not when cutting open the tissues up to the osseous tissue after cutting open the skin, etc., by extracting the image of the important organ, such as, a tendon in the vicinity of the bone of the surgical field, etc., for example, within the image extracting unit 102, and displaying it/putting them on the image of the bone. Also, in the image processing unit 103, it is possible to make a confirmation on the relationship between the Ostectomy Lines and the important organ(s), while deleting or moving the tissues in front of the Ostectomy Lines within the image extracting unit 102.

The correction of the Ostectomy Line can be done by clicking the mouse at the reference point on the stereographical view, while pushing down the button at the reference point on the dialog screen shown in Fig. 4, on which the correction wished to be made, thereby clicking the mouse at the reference points on the stereographic image again. After completing the correction, when the decide button 131 is pushed down on the dialog screen 132 shown in Fig. 4 by the surgical doctor, the Ostectomy Line(s) is/are presented to the surgical doctor, on which the spherical surface passing through the four (4) reference points comes crossing with the bone.

The navigation system 3 comprises: a marker 300; a three-dimensional position measurement apparatus 301; a position information integration unit 302; an image pick-up device 100; an image producing unit 101; an image extracting unit 102; an image processing unit 103; and an image displaying apparatus 104. However, the image pick-up device 100, the image extracting unit 102, the image processing unit 103 and the image displaying apparatus 104 are used in common with the surgical field shape grasping function unit 10, as shown in Fig. 1.

The navigation system 3 obtains a conversion matrix for converting the position coordinate system of an actual patient into the position coordinate system of the medical use image of the patient, which was picked up before making up the surgical operation plan, in the position information integration unit 302 thereof, and it multiplies the position coordinates of the surgical operation tool onto the coordinate system of the actual patient with that conversion matrix obtained, thereby obtaining the coordinates of the surgical operation tool on the coordinate system of the medical use image of the patient. Thereafter, it produces an image for presenting the surgical operation tool on the medical use image of the patient, which is picked up before making up the surgical operation plan, in the image producing unit 101, and it produces an image, laying that image on the surgical operation route, which was produced in the surgical operation route calculation unit 107, within the image processing unit 103, thereby presenting them to the surgical doctor on the image displaying apparatus 104 on a real-time basis.

The surgical operation robot 2 comprises: a manipulator (i.e., a robot arm) 201 for holding the surgical operation tool thereon, and a manipulator controller apparatus 200 for operating it. The operation of the surgical operation robot 2 can be selected to operate automatically or manually. When operating the surgical operation robot 2 automatically, the result calculated in the surgical operation route calculation unit 107 is transmitted to the manipulator controller apparatus 200, so as to perform the surgical operation while controlling the manipulator 201 in accordance with the surgical operation route planned.

When operating the surgical operation robot 2 manually, the surgical operation is conducted through operation of the manipulator 201 while transmitting the control information from an operation table to the manipulator controller apparatus 200. However, when performing the surgical operation manually, it is conducted while confirming the shifting between the surgical operation plan and the actual surgical operation, with using the navigation system 3.

Herein, to be the medical use image of the patient picked up before making up the plan of surgical operation, the medical use image is used, which was used in the surgical operation planning system 1. However, the image was picked up under the condition that at least three (3) markers or more are attached thereupon, when being picked up at first by means of the image pick-up device 100. The conversion matrix, for converting from the position coordinate system of the actual patient into the position coordinate system on the medical use image of the patient, which was picked up before making up the surgical operation plan, can be obtained in the following manner. Thus, first of all, the same number of the markers 300 are attached on the actual patient at the positions where the markers of a number of at least three (3) or more are attached, when picking up the medical use image of the patient before making up the surgical operation plan, and the position coordinates of those markers are measured through the three-dimensional position measurement apparatus 301, and thereby indicating them by means of a matrix of 3x3, or more than that. A matrix for converting that matrix into the matrix of 3x3 or more than that for representing the marker position coordinates on the medical use image of the patient, which was picked up before making up the surgical operation plan, is obtained through calculation conducted within the position information integration unit 302.

The position coordinates of the surgical operation tool can be obtained on the position coordinate system of the actual patient, as will be mentioned below. First of all, the three (3) reference points of the surgical operation robot 2 are measured by means of the three-dimensional position measurement apparatus 301 on the position coordinate system of the actual patient. Next, in the position information integration unit 302, from the designed numeral values of the surgical operation robot 2 and the reference position of the surgical operation robot 2 that is obtained from the control information outputted from the manipulator controller apparatus 200, the conversion matrix up to the surgical operation tool held at a tip of the manipulator is multiplied onto the coordinates of the three (3) reference points of the surgical operation robot 2 in the position coordinate system of the actual patient. With this, the position coordinates of the surgical operation tool mentioned above can be obtained.

In the position information integration unit 302, inputs of the positions of the markers on the medical use image and the positions of the markers 300 attached on the actual patient as well as inputs of the reference points of the surgical operation robot are treated in the following manner.

When selecting "Navigation Initial Setting" from the menu-bar on the image display apparatus 104, the dialog screen 314, such as shown in Fig. 5, appears at an end portion of the screen. The surgical doctor conducts the inputting after completing selection of one to be inputted, from a tab "Medical Use Image" 310, a tab "Actual Patient" 311, and a tab "Surgical Operation Robot" 312, which are displayed on the dialog screen 314.

Herein, when inputting the position of the markers on the medical use image, after selection of the tab "Image For Medical Use" 310 in Fig. 5, the stereographic image is rotated freely on the screen of the medical use image through dragging the mouse, thereby displaying an arbitrary point, at which an input is desired to be made, as an input point. Under this condition, a first one is inputted through clicking of the mouse at the arbitrary point. When correcting, it is achieved by clicking the mouse again, after pushing down the correct button 313 at the point corresponding thereto on the dialog screen 314 shown in Fig. 5. In a case of designating a second one and also others thereafter, the point where the mouse is clicked is automatically acknowledged to be a next input point, when clicking the mouse, but without pushing down the correction button after designating the last input point.

When inputting the positions of the markers 300 on the actual patient, after selecting the tab "Actual Patient" 311 in Fig. 5, the position information of the markers are transmitted to the position information integration unit 302, while putting a measuring probe of the three-dimensional position measurement apparatus 301 on the markers 300 attached on the patient, in the same order or sequence when inputting the positions of the markers on the medical use image. When completing the transmission thereof, since the position coordinate numerical values are displayed, such as, like the screen shown in Fig. 6, an input button 315 of the corresponding marker is pushed down after confirming the display thereof. When designating a second input point and also others thereafter, after designating the last input point, the measuring probe of the three-dimensional position measurement apparatus 301 is also put on the marker 300 attached on the patient, but without pushing down a correct button 316. With this, the position information of the marker 300 is transmitted to the position information integration unit 302, and the position coordinate numerical values 314a are displayed in a similar manner when inputting the position information of the first marker, therefore the input button 15 can be pushed down after confirming the display thereof. However, when correcting, it can be made by putting the measuring probe of the three-dimensional position measurement apparatus 301 on the markers attached on the patient, again, after pushing down the correct button at the corresponding point on the dialog screen shown in Fig. 6.

When inputting the reference points of the surgical operation robot, the operation is conducted, as will be explained in the following. When selecting the tab "Surgical Operation Robot" 312 on the dialog screen shown in Fig. 5, a marker position input screen for the surgical operation robot is displayed in a similar manner to the marker position input screen of the actual patient shown in Fig. 6. On that screen, the measuring probe of the three-dimensional position measurement apparatus 301 is put on the reference points, in accordance with a predetermined order or sequence thereof. With this, the position information of the reference points are transmitted to the position information integration unit 302, and the position coordinate numerical values 314 are displayed, in the similar manner to the dialog screen shown in Fig. 6, therefore the input button 315 can be pushed down after confirming the display thereof. However, when making correction, it is only possible by putting the measuring probe of the three-dimensional position measurement apparatus 301 on the reference points, again, but after pushing down the correct button 316 at the corresponding point on the dialog screen, which is same to that when inputting.

Since the dialog screen is displayed for the purpose of confirming the completion of all the inputs, when inputting all of the position coordinates of all points, such as, the positions of the markers on the medical use image, the position of the markers attached on the actual patient, and the reference points of the surgical operation robot, etc., then the input can be completed by selecting "OK" on this dialog screen.

The screen, for presenting the position of the surgical operation tool on the medical use image of the patient that was picked up before making up the surgical operation plan, is selectable from among a plural number of screens. Namely, either one can be selected by the surgical doctor: e.g., a screen of displaying 3-D images of the surgical operation robot and the surgical operation tool, being piled up on the medical use image of the patient that was picked up before making up the surgical operation plan; or, a screen displaying a 3-D image of only the surgical operation tool, being piled up on the medical use image of the patient that was picked up before making up the surgical operation plan. In a method for that selection, selection is made on either "Robot and Tool" or "Only Tool" from the menu-bar, after selecting "Type Selection of Navigation Image". However, this selection can also be made during conduction of the surgical operation. And also, the position coordinates of the surgical operation tool within the position coordinate system of the actual patient may be obtained, through measuring the positions of the markers, which are directly attached on the surgical operation tool, by using the three-dimensional position measurement apparatus 301.

As a method for presenting the position of the surgical operation tool(s) on the medical use image of the patient, which was picked up before making up the surgical operation plan, the images of all the surgical operation tools to be used are picked up, before conducting the surgical operation by means of the image pick-up device 100, and this 3-D image obtained is displayed, while piling it up on the medical use image of the patient. However, the 3-D image of the surgical operation robot is inputted into a film, which was taken in advance. As a method for selecting the image of the surgical operation tool, every time when changing the surgical operation tool, "Selection of Tool File" is selected from the menu-bar, and then the tool file is selected from a dialog screen for opening the file.

According to the present embodiment, in the stage of planning the surgical operation, it is possible to provide the surgical operation assistance system, enabling to draw a smooth surgical operation route, being necessary, in particular, in the ostectomy, such as, the RAO with the aid of a surgical operation robot, etc., for example. Also, since the surgical operation route can be determined to be a smooth surface, and not an aggregation of lines, therefore input items, being necessary when determining the smooth surgical operation route, come to be less in the number thereof than that of a method of determining inflection points on the way thereof, thereby obtaining elimination or reduction of labors for the surgical doctor, and further, it is possible to provide the surgical operation assistance system to be applicable in orthopedic surgery, in general.

Next, explanation will be given on a second embodiment according to the present invention ,which is applied when conducting a surgical operation other than that mentioned above, i.e., by means of the RAO with using the surgical operation robot.

In this second embodiment, a reference point input unit is provided for the purpose of inputting the kind of the surgical operation, such as, the ostectomy or drilling, for example, in an input format of a button, a slider, a menu-bar, or others, displayed on the screen; or, for the purpose of inputting the kind of lines for use of the approximation, in an input format of the button, the slider, the menu-bar or others, displayed on the screen, in particular, on the surgical operation, in which a portion of the surgical operation route can be approximated by an ellipse, a cylindroid, a circle, a column, a parabola, a parabolic cylinder, a straight line, a rectangular, a parallelepipedon, or a sphere. Also, the 3-D position coordinates of the number of reference points, being necessary for corresponding to the kind of lines for the approximation, can be designated by clicking of the mouse or a touch panel, on the stereographic medical use image of the patient, which was picked up before the surgical operation.

As is apparent from the explanation given on various embodiments in the above, according to the present invention, it is possible to easily determine a smooth surgical operation route to the surgical field, by means of the surgical operation assistance system and the surgical operation assisting method, and also the software thereof, enabling easy orthopedic surgical operations, with certainty and safety.

The present invention may be embodied in other specific forms without departing from the spirit or the essential features or characteristics thereof. The present embodiment(s) is/are therefore to be considered in all respects as only illustrative and not restrictive, the scope of the invention being indicated by the appended claims rather than by the foregoing description, including the range of equivalency of the claims.

### List of Reference Signs

- 1: surgical operation planning system
- 2: surgical operation robot
- 3: navigation system
- 4: surgical operation assistance system

- 10: surgical field shape grasping function unit
- 11: surgical operation route determining function unit

- 100: image pick-up device
- 101: image producing unit
- 102: image extracting unit
- 103: image processing unit
- 104: image displaying apparatus
- 105: slice image arbitrary line input unit
- 107: surgical operation route calculation unit
- 108: reference point input unit

- 110: acetabular roof
- 111: defective portion
- 112: bone cutting line
- 113: caput ossis femoris

- 120: correct button
- 121: decide button
- 122: correct button
- 123: dialog screen

- 130: correct button
- 131: decide button
- 132: dialog screen

- 200: manipulator controller apparatus
- 201: manipulator
- 300: marker
- 301: 3-D position measurement apparatus
- 302: position information integration unit

- 310: medical use image tab
- 311: actual patient tab
- 312: surgical operation robot
- 313: correct button
- 314: dialog screen
- 314a: position coordinate numerical values
- 315: input button
- 316: correct button

## Claims

1. Surgical operation assistance system (4), comprising:
- an image pick-up device (100) for picking up an image of a surgical field,
- an image producing unit (101) for producing a stereographic image of the surgical field, the image of which is picked up,
- a reference point input unit (108) for inputting reference points of a surgical operation route based on the kind of the surgical operation and the stereographic image,
- a surgical operation route calculation unit (107) for calculating a smooth surgical operation route based on the kind of the surgical operation inputted and the reference points,
- an image processing unit (103) for processing the stereographic image and the surgical operation route to be displayable, and
- an image displaying apparatus (104) for displaying the images processed in the image processing unit (103) thereon.

2. Surgical operation assistance system according to claim 1, further comprising an image extracting unit (102) for extracting a partial image from the stereographic image, wherein the image processing unit (103) processes the extracted image to be displayable.

3. Surgical operation assistance system according to claim 1 or 2, further comprising a slice image arbitrary line input unit (105) for designating an arbitrary line of a sliced image to be displayed, wherein the image processing unit (103) processes the sliced image to be displayable, based on the arbitrary line designated for the slice image.

4. Surgical operation assistance system according to any of claims 1 to 3, further comprising a surgical operation robot (2) for automatically conducting the surgical operation on the surgical field with using the surgical operation tool along with the calculated surgical operation route.

5. Surgical operation assistance system according to any of claims 1 to 4, further comprising a slice image arbitrary line input unit (105) for designating an arbitrary line of a sliced image to be displayed, wherein the image processing unit (103) processes the sliced image to be displayable; based on the arbitrary line designated for the slice image.

6. Surgical operation assistance system according to claim 4 or 5, comprising a navigation system (3) which comprises a position information integration unit (302) for integrating position information of the surgical robot (2) with an image of the surgical field picked up by the image pick-up device (100).

7. Surgical operation assistance system according to any of claims 1 to 6, further comprising:
- an image producing unit (101) for producing a stereographic image of the surgical field picked up, and for producing an image piling up an image of the surgical operation tool on the stereographic image, based on the information integrated in the position information integration unit (302).

8. Surgical operation assistance system according to claim 6 or 7, wherein the navigation system (3) further comprises a marker (300) and a 3-D position measurement apparatus (301).

9. Surgical operation assistance system according to any of claims 4 to 7, wherein the surgical operation robot (2) is also operable manually on the surgical field, with using the surgical operation tool along with the calculated surgical operation route, and the surgical operation robot (2) is switchable between automatic operation and manual operation.

10. Surgical operation assistance system according to any of claims 1 to 9, which is designed to implement the following operational steps:
- picking up an image of a surgical field,
- producing a stereographic image of the surgical field,
- inputting reference points of a surgical operation route based on the kind of the surgical operation and the stereographic image,
- calculating a smooth surgical operation route based on the kind of the surgical operation inputted and the reference points,
- processing the stereographic image and the surgical operation route to be displayable, and
- displaying the processed images.

11. Surgical operation assistance system according to any of claims 1 to 10, which is designed to implement the following operational steps:
- picking up an image of a surgical field,
- conducting a surgical operation manually on the surgical field with using a surgical operation tool of a surgical operation robot,
- integrating position information of the surgical operation robot with an image of the surgical field picked up,
- producing a stereographic image of the surgical field picked up, and producing an image piling up an image of the surgical operation tool on the stereographic image, based on the integrated information,
- inputting reference points of a surgical operation route based on the kind of the surgical operation tool and the stereographic image,
- calculating a smooth surgical operation route based on the kind of the surgical operation tool and the inputted reference points,
- processing the stereographic image and the surgical operation route to be displayable under a desired condition, and
- displaying the processed image.

12. Surgical operation assisting method, comprising the following steps:
- picking up an image of a surgical field by means of an image pick-up device,
- producing a stereographic image of the surgical field, the image of which is picked up in an image producing unit,
- inputting reference points of a surgical operation route based on the kind of the surgical operation and the stereographic image through an input unit,
- calculating a smooth surgical operation route based on the kind of the surgical operation inputted and the reference points in a surgical operation route calculation unit,
- processing the stereographic image and the surgical operation route to be displayable in an image processing unit, and
- displaying the images processed in the image processing unit on an image displaying apparatus.

13. Surgical operation assisting method preferably according to claim 12, comprising the following steps:
- picking up an image of a surgical field by means of an image pick-up device,
- conducting a surgical operation manually on the surgical field with using a surgical operation tool of a surgical operation robot,
- integrating position information of the surgical operation robot with an image of the surgical field picked up by the image pick-up apparatus in a position information integration unit,
- producing a stereographic image of the surgical field picked up, and producing an image piling up an image of the surgical operation tool on the stereographic image, based on the information integrated in the position information integration unit, in an image producing unit,
- inputting reference points of a surgical operation route based on the kind of the surgical operation tool and the stereographic image through a reference point inputting unit,
- calculating a smooth surgical operation route based on the kind of the surgical operation tool and the inputted reference points, in a surgical operation route calculating unit,
- processing the stereographic image an the surgical operation route to be displayable under a desired condition in an image processing unit, and
- displaying the image processed in the image processing unit on an image displaying apparatus.

14. Surgical operation assisting method, according to claim 12 or 13, wherein the image of the surgical field is picked up by means of the image pick-up device under a condition where three (3) or more markers are attached thereon, thereby producing a medical use image, further comprising the following steps:
- attaching the same number of markers on an actual patient at positions where the markers are attached, measuring the position coordinates of those markers through a 3-D position measurement apparatus, thereby presenting them in a form of a matrix of 3x3 or more, converting this matrix into a matrix of 3x3 or more for presenting the position coordinates of the markers on the medical use image in the position information integration unit, and producing an image piling up the image of the surgical operation tool on the stereographic image, based on the converted matrix.

15. Program stored on a computer-readable storage medium for assisting surgical operations, comprising the following steps:
- a step of picking up an image of a surgical field by means of an image pick-up device,
- a step of producing a stereographic image of the surgical field, the image of which is picked up, in an image producing unit,
- a step of inputting reference points of a surgical operation route based on the kind of the surgical operation and the stereographic image through an input unit,
- a step of calculating a smooth surgical operation route based on the kind of the surgical operation inputted and the reference points in a surgical operation route calculation unit,
- a step of processing the stereographic image and the surgical operation route to be displayable in an image processing unit, and
- a step of displaying the images processed in the image processing unit on an image displaying apparatus.

16. Use of the surgical operation assistance system according to any of claims 1 to 11 and/or application of the surgical operation assistance method according to any of claims 12 to 15 for orthopedic surgical operations.
